(19) Europäisches Patentamt
European Patent Office
Office européen des brevets

(11) Publication number: **0 066 379**
**A2**

# (12) EUROPEAN PATENT APPLICATION

(21) Application number: 82302348.6

(22) Date of filing: 07.05.82

(51) Int. Cl.³: A 61 K 31/72
C 08 B 37/02

(30) Priority: 15.05.81 US 263987

(43) Date of publication of application: 08.12.82 Bulletin 82/49

(84) Designated Contracting States: BE CH DE FR GB IT LI NL SE

(71) Applicant: RIKER LABORATORIES, INC.
19901 Nordhoff Street
Northridge California(US)

(72) Inventor: Miller, Richard L.
2501 Hudson Road P.O. Box 33427
St. Paul Minnesota 55133(US)

(72) Inventor: Mosbey, Deral T.
2501 Hudson Road P.O. Box 33427
St. Paul Minnesota 55133(US)

(74) Representative: Baillie, Iain Cameron et al,
c/o Ladas & Parry Isartorplatz 5
D-8000 München 2(DE)

## (54) Composition for combating herpes virus.

(57) A composition for combatting herpes simplex viruses types I and II, comprising a dextran sulfate sodium salt having a weight average molecular weight from about 4,100 to 25,000 and containing at least about 0.2 equivalent of $SO_3Na$ per glucopyranosyl unit therein.

EP 0 066 379 A2

# COMPOSITION FOR COMBATTING HERPES VIRUS

## Technical Field

The present invention relates to a composition for combatting types I and II herpes simplex virus.

## Background Art

Although herpes infections have been a difficult therapeutic problem for many years, safe and effective treatments are not currently available. The present treatments for acute herpes infections, such as 5-iodo-2'-deoxyuridine and 9-D-arabinofuranosyl adenine have potentially serious side effects and are not generally used to treat mild herpes infections.

Dextran sulfate polymers are known as are various biological effects thereof. For example, very high molecular weight dextran polymers have been reported by Nahmias _et. al._, Proc. Soc. Expt. Biol. Med. _115_, 993-6 (1964) to possess anti-Herpes activity. Similarly Frank _et. al._, Arch, Virol. 58: 259-268 (1978), have reported anti-Herpes activity for dextran sulfate polymers having molecular weights of 500,000. However, such high molecular weight polymers appear to have unacceptable short and/or long term toxicology problems and have never been made commercially available as antivirals.

Low molecular weight dextran sulfate polymers have also been tested for biological activities. For example, Burger, _et. al._, Immunology, 1975, _29_, 549, found that dextran sulfates of molecular weight of 10,000, 80,000, 250,000 and 2,000,000 were activators of the alternate pathway of complement when sufficiently highly sulfated. Ricketts, _et. al._, Chemistry and Industry, September 6, 1952, p. 869 describes the investigation of the anticoagulant properties of dextran sulfates of molecular weight about 7,300.

However, no reports of antiviral activity of dextran sulfates of molecular weight between 4,000 and 25,000 have appeared prior to the present invention, let alone anti-herpes activity. The general teaching of the art has, in fact been that decreased molecular weights in polymeric drugs are associated with reduced antiviral activity. For example, the recent publication of the International Symposium on Polymeric Drug, New Orleans, 1977 entitled "Polymeric Drugs," edited by Donaruma and Vogel and published in 1978 by Academic Press states on page 277: "The clinical future of polyanions lies with the separation of toxicity from antitumor, antiviral, RES, and immunologic effects. It is apparent from data so far that in general the toxicity of polyanions increases with molecular weight particularly over 50,000. Fortunately antitumor activity is retained in lower MW fractions (<10,000). These fractions are relatively less toxic and are associated with macrophage activation. However, they are devoid of antiviral activity. It may be more difficult to separate toxicity from antiviral or immunologic stimulating effects that apparently reside in molecular weights above 30,000." Thus, low molecular weight polyanionic polymeric drugs are generally considered uninteresting and inactive as antiviral agents.

Disclosure of Invention

The present invention relates to a composition for controlling Type I or Type II Herpes simplex virus comprising a dextran sulfate sodium salt having a weight average molecular weight from about 4,100 to 25,000 and containing at least about 0.2 equivalent $-OSO_2Na$ (i.e. $SO_3Na$) per glucopyranosyl unit therein.

At molecular weights above and below this range, the antiviral activity of polymers of this type is negligible as is that of unsulfated dextrans within this

range of molecular weights. The preferred molecular weight range of the dextran sulfate sodium salts is 4,100-18,000 since the antiviral activity is greater in this range. More preferably, the weight average molecular weight of the salt is from about 9,000 to 16,000 and it contains at least about 0.4 equivalent of $SO_3Na$ per glucopyranosyl unit.

Dextrans are polysaccharides varying in molecular weight and branching and composed of α-D-glucopyranosyl units

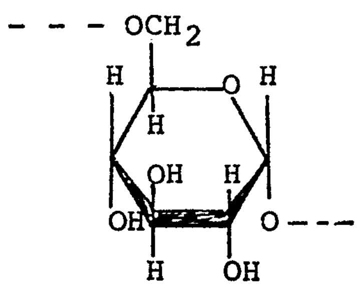

I

The three hydroxyl groups on each such unit are capable of sulfation (i.e. conversion to the sulfate $-OSO_2H$) and that group in turn can be converted to salt form $-OSO_2Na$. Thus, a completely sulfated glucopyranosyl unit would contain three sulfate groups.

The dextran sulfate sodium salt can be used *in vitro* (for controlling active virus in laboratory, medical and veterinary facilities) and *in vivo* for treating infections caused by the viruses (especially in mammals). By active virus is meant non-dormant virus. The dextran sulfate sodium salt is effective when administered subcutaneously, intravenously, or intraperitoneally. Suitable pharmaceutically acceptable carriers for the dextran sulfate sodium salt include such conventional carriers as 0.9 N saline solution and an aqueous solution of acacia.

The dextran sulfate salts are conveniently prepared by sulfation of a corresponding dextran in the presence of a strong organic base solvent and a lower alkanoic amide followed by the reaction of the resulting

alkanoic amide followed by the reaction of the resulting product with sodium hydroxide to form the salt. Suitable precursor dextrans are available or can be prepared by known processes. There is little or no degradation of the dextran during the reaction and the nature of the precursor itself and the degree of sulfation achieved are therefore strongly determinative of the nature of the final salt. The sulfation is carried out by means of a reactive sulfating agent such as chlorosulfonic acid. Suitable solvents include pyridine, quinoline, triethylamine and the like. The preferred lower alkanoic amide is formamide.

The sulfated polymer is separated from both the organic solvent and the residual chloride ion and converted to the salt (suitably by reaction with sodium hydroxide). The reaction conditions for the sulfation reaction require mild heating e.g. 50° to 60°C with careful control of the exothermic reaction. Degrees of sulfation up to about 2.4 equivalents per glucopyranosyl unit can be conveniently achieved by this process.

The values obtained for the molecular weights of polymers of the type utilized in the present invention vary to some extent with the particular test method used, and it is therefore necessary in referring to test results, ranges of values, etc. to specify the method (and also the test conditions). The high pressure liquid chromatography method described by Petracek and Sugisaka in the Federation Proceedings, Vol. 36, No. 1, January 1977, pages 89-92, has been found to be particularly dependable for determining the molecular weights of these polymers and is therefore utilized herein. It is sometimes referred to as HPLC, although frequently no particular designation is given.

Both 0.5 molar aqueous sodium sulfate and 1 or 1.5 molar aqueous sodium chloride have been used as the mobile phase in carrying out the HPLC molecular weight determinations, and other salts and other concentrations could be used as well. Although the absolute values

obtained using the sodium sulfate and sodium chloride systems do differ, there is a definite relationship between them and a measured value in one system can be readily converted into the other. There is no substantial difference in the absolute values obtained using 1 or 1.5 molar sodium chloride, however. The 1 or 1.5 molar sodium chloride system is presently preferred and the values herein are given in terms of that mobile phase and using dextrans as the standard.

Three complementary test values, all based upon HPLC data (and all being well known to those skilled in the art), are utilized. Thus, the weight average molecular weight (Mw) is perhaps the best single value of the molecular weight of a polymer sample and the ratio of the weight average molecular weight to the number average molecular weight (Mn), conventionally referred to as polydispersity (D), is a generally recognized measurement of the overall sharpness or breadth of the range of the molecular weights of the polymer chains therein.

The dextran sulfate sodium salts can be fractionated using ultrafiltration to provide samples with narrower molecular weight ranges, if desired.

## Detailed Description

The following examples illustrate the preparation of the dextran sulfate sodium salts.

The microtiter plate method (described by John A. Montgomery, Anita T. Shortnacy, G. Arnett and W. M. Shannon in the Journal of Medicinal Chemistry, Vol. 20, No. 30, pp. 401-404 (1977) is applied in the tests described in the examples to the standard Vero cell antiviral bioscreen (described by P. Collins and D. J. Bauer, in Annals of the New York Academy of Sciences, Vol. 284 (1977) at pages 49-59) to determine the antiviral activity of the dextran sulfate sodium salts *in vitro*.

Example 1

Dry pyridine (140 ml) is maintained below 20°C by external cooling while adding dropwise 16.7 ml (29 g, 250 mmole) of chlorosulfonic acid. To this mixture is added 10 ml of formamide. The mixture is stirred for ten minutes, then 6.8 grams of a dextran polymer having an average molecular weight of about 10,000 ("Dextran T-10" available from Pharmacia Fine Chemicals of Piscataway, N.J.) is added. A white solid precipitates and the mixture is stirred with difficulty while warming to 55° to 60°C. The temperature reaches a maximum of 70°C, but immediately cools to 60°C. The mixture is stirred for 6 hours. The solvent is then removed by decantation and the residue is gradually dissolved in 70 ml of water. The product is precipitated by pouring the aqueous solution into 500 ml of methanol. The solvent is again removed by decantation and the product residue dissolved in 50 ml of water. The product is precipitated by pouring the aqueous solution into 500 ml of isopropanol. The solvent is removed by decantation and the residue is dissolved in 50 ml of water. The solution is basified with 50% sodium hydroxide solution, then poured into 500 ml of methanol. To this mixture is added 300 ml of diethyl ether. The solid is separated by filtration and dissolved in 100 ml of water. The resulting solution is dialyzed until all chloride is removed (i.e. until no precipitate forms when silver nitrate is added), then lyophilized and dried in vacuo. The product is found to contain 19.0% sulfur. The weight average molecular weight (Mw) when measured by high pressure liquid chromatography is 15,200 and the polydispersity (D) is 1.40. The yield of product is 12.0 g of the sodium salt of sulfated dextran.

Thus, in Example 1, approximately a 100% excess of chlorosulfonic acid is charged (sufficient to provide six equivalents of sulfate for the three possible hydroxy reaction sites in each α-D-glucopyranosyl unit in the dextran starting material) and, as calculated from the

percent sulfur in the product, a degree of substitution of about 2.4 equivalents of sulfation per glucopyranosyl unit results (80% of the theoretical maximum).

This reaction is repeated twice using the same amounts of reagents. Temperature control is improved as shown:

| Example No. | Product (g) | Maximum Temp (°C) | Percent S | Degree of $SO_3Na$ Substitution (1) | $\bar{Mw}$ | D |
|---|---|---|---|---|---|---|
| 2 | 14.4 | 64 | 18.4 | 2.25 | 15,600 | 1.38 |
| 3 | 13.0 | 60 | 18.7 | 2.34 | 15,700 | 1.38 |

(1) Equivalents of $SO_3Na$ per glucopyranosyl unit

## Example 4

A 25 g sample of dextran sulfate sodium salt (prepared as above from "Dextran T-10") is dissolved in 200 ml of 10% sodium chloride solution. The mixture is ultrafiltered on a PM10 membrane at 50 psi and, after 100 ml of filtrate is collected, 100 ml of 10% sodium chloride solution is added to the retentate. This process is repeated until 500 ml of filtrate (A) has been collected.

The remaining 100 ml of retentate is transferred to a cell fitted with a PM 20 membrane. To the retentate is added 100 ml of 10% sodium chloride solution. Filtration is carried out repeatedly as above until 500 ml of filtrate (B) has been collected.

The remaining 100 ml of retentate is transferred to a cell fitted with a PM 30 membrane. To the retentate is added 100 ml of 10% sodium chloride solution and filtration is carried out repeatedly as above until 500 ml of filtrate (C) has been collected. The remaining 100 ml of retentate is considered fraction (D).

Each of the fractions, A, B, C and D, is placed in a dialysis bag and dialyzed until the water washes are free of chloride ion (no precipitation with silver nitrate). This requires 15 to 30 hours and many washes.

Each sample is then lyophilized. The results are shown in the following table:

| Sample | Product (g) | Percent S | Degree of $SO_3Na$ Substitution (1) | $\overline{Mw}$ | D |
|---|---|---|---|---|---|
| Starting Material | | | | 12,900 | 1.52 |
| A | 7.2 | 18.7 | 2.34 | 9,000 | 1.40 |
| B | 3.5 | 18.2 | 2.19 | 14,400 | 1.50 |
| C | 11.1 | 18.7 | 2.34 | 16,000 | 1.39 |
| D | 0.3 | (2) | (2) | 16,000 | 1.36 |
| | 22.1 Total (88% recovery) | | | | |

(1) Equivalents of $SO_3Na$ per glucopyranosyl unit

(2) Not measured

Example 5

The antiviral activity of the low molecular weight dextran sulfate sodium salt of Example 1 is measured in vitro against herpes simplex virus type I KOS, herpes simplex virus type I B2006 which lacks the virus induced thymidine kinase and herpes simplex virus type II 333 utilizing the Vero cell microtiter plate method referred to previously. The minimal inhibitory concentration measured is 2.5 g/ml against each of these strains, yet the salt shows no toxicity (no effect on Vero cell growth) at 100 to 400 g/ml.

Example 6

The effect of the degree of sulfation upon the activity of the dextran sulfate sodium salts against herpes simplex virus type I is determined, utilizing the Vero cell microtiter plate method. The starting material and process of Example 1 is used to prepare the salts, but the proportions of the chlorosulfonic acid to the dextran is varied to obtain products with varying degrees of

sulfation. The products are then evaluated for their antiviral actitivy with the following results:

| | Equivalents of $SO_3Na$ per Glucopyranosyl Unit | | Antiviral Activity, |
|---|---|---|---|
| Lot | In the Charge | In the Product | Minimum Inhibitory Concentration ( g/ml) |
| A | 6 | 1.9-2.3 | 2.5 |
| B | 2.3 | 1.12 | 2.5 |
| C | 2.0 | 0.86 | 5.0 |
| D | 1.7 | 0.63 | 5.0 |
| E | 1.4 | 0.48 | 5.0 |
| F | 1.1 | 0.13 | >100 |

Thus, utilizing this process, the charge must contain a considerable excess of chlorosulfonic acid in order to approach the theoretical maximum of three $SO_3Na$ groups per glucopyranosyl unit in the product. However, a high degree of antiviral activity is maintained in the polymers with as few as about 0.2 equivalents of $SO_3Na$ per glucopyranosyl unit.

## Example 7

The antiviral activity of the dextran sulfate sodium salts is evaluated *in vivo* utilizing eighteen day old Swiss mice as the test animals. The product salt and dextran starting material of Example 1 are compared and the tests are carried out as follows:

Infection of the mice. Intraperitioneal (IP) injection with 100 plaque-forming units of herpes simplex virus type I (a normally lethal challenge).

Administration of the drugs. The drugs are suspended in 4% acacia and administered IP twice daily for four days beginning one one hour before infection.

Dosage. The dosages are reported as milligrams of the drug per kilogram of body weight in each administration (mg/kg/admin). One group of eight mice receives

10 mg/kg/admin of sodium dextran sulfate and two other groups of eight mice each receive 1 mg/kg/admin and 0.1 mg/kg/admin of sodium dextran sulfate. One group of 9 mice receives 10 mg/kg/admin of dextran and another (control) group of 43 mice receives no drug.

Duration of the test. Survival is noted for twenty days.

The results are given in the following table in which Drug I is the dextran sulfate sodium salt product of Example 1, Drug II is the dextran starting material of Example 1, Results A report the number of mice surviving in the group (at the end of the twenty day observation period of the test)/the total number of mice in the group and Results B report the mean survival time in days of the group:

| | | Dosage (mg/kg/admin) | | | |
|---|---|---|---|---|---|
| Drug | Results | 10 | 1 | 0.1 | 0 |
| I | A | 5/8 | 6/8 | 0/8 | 2/43 |
| I | B | 15.1 | 16.5 | 6.1 | 6.9 |
| II | A | 0/9 | (1) | (1) | 2/43 |
| II | B | 6.4 | (1) | (1) | 6.9 |

(1) Not measured

Thus, sulfated polymer increases survival at 10 and 1 mg/kg/admin while dextran does not increase survival. Sulfated polymers of weight average molecular weight ($\overline{Mw}$) from 4,100 (D 1.3) to 18,000 increase survival in this test model when administered intraperitoneally at 1 mg/kg/admin.

Example 8

Dextran sulfate sodium salt within the molecular weight range 4,100 to 18,000 is also found to reduce cutaneous herpes simplex virus type I lesions in guinea pigs. Thus, areas on the midbacks of male Hartley guinea pigs are depilated. Twenty four hours later, skin in

these areas is infected using a multiple puncture vaccination device. The dextran sulfate sodium salt is suspended in 4% acacia and delivered intraperitoneally utilizing the same schedule as in Example 7, but beginning one hour after infection. No lesions develop in animals that receive 50 mg/kg/admin IP and lesions are reduced in those that received 5 or 10 mg/kg/admin. No therapeutic activity is observed when the polymers are administered topically in this model.

Example 9

The acute IP $LD_{50}$ of the dextran sulfate sodium salts utilized in the processes of the invention is found to be approximately 170 mg/kg in rats.

Claims:

1. A composition for controlling Type I or Type II Herpes simplex virus, comprising a dextran sulfate sodium salt having a weight average molecular weight from about 4,100 to 25,000 and containing at least about 0.2 equivalent of $SO_3Na$ per glucopyranosyl unit therein.

2. A composition according to claim 1, wherein said salt has a weight average molecular weight of from about 4,100 to 18,000.

3. A composition according to claim 1, wherein said salt has been prepared by sulfating a suitable low molecular weight dextran in the presence of a strong organic base solvent and a lower alkanoic amide and reacting the resulting product with sodium hydroxide.

4. A composition according to claim 3, wherein said sulfation is carried out at from about 50° to 60°C in the presence of pyridine and formamide.

5. A composition according to claim 1, wherein said salt contains from about 0.4 to 2.4 equivalents of $SO_3Na$ per glucopyranosyl unit therein.

6. A composition according to claim 1, wherein said salt has a weight average molecular weight from about 9,000 to 16,000.

7. A composition according to claim 1, further comprising a pharmaceutically acceptable carrier for said salt.

8. A composition as defined in any one of claims 1 to 7 for use in a method of controlling Type I or Type II Herpes simplex virus.